# EUROPEAN PATENT APPLICATION

(11) **EP 1 234 886 A1**
(43) Date of publication of application: **28.08.2002**
(21) Application number: 01200701.9
(22) Date of filing: 23.02.2001
(51) Int. Cl.: C12Q 1/48, C12Q 1/18, C12N 9/10

(54) **A method for detecting beta-1,6-glucan synthase activity**

(71) Applicant: Universiteit van Amsterdam, 1012 WX Amsterdam (NL)
(72) Inventor: Klis, Frans, 1012 WX Amsterdam (NL)
(74) Representative: Prins, Adrianus Willem

(57) **Abstract**

The invention relates to the field mycology, in particular anti-fungal development, and medicine. In one aspect the invention provides a method to for identifying a substance capable of interacting with beta-1,6 glucan synthase comprising contacting a substrate for beta-1,6 glucan synthase with a material comprising beta-1,6 glucan synthase activity in the presence of said substance under appropriate reaction conditions for said synthase and determining whether beta-1,6 glucan synthase activity is afffected by the presence of said substance. In another aspect the invention provides a high throughput screen for the identification of said substance, comprising an inhibition Immunoassay, wherein said immunoassay comprises contacting a reaction product, obtained by said contacting of a beta-1,6 glucan synthase substrate with said synthase in the presence of said substance, with beta-1,6 glucan coated onto a solid surface and antibody with binding specificity for beta-1,6 glucan and determining antibody bound to said solid surface coated with beta-1,6 glucan and/or antibody not bound to said solid surface coated with beta-1,6 glucan.

## Description

The invention relates to the field mycology, in particular anti-fungal development and medicine.

Fungi have become an increasing threat to humans. Opportunistic fungal infections have increased dramatically in the last two decades and have become a significant cause of morbidity and mortality. Over recent years, the frequency of life-threatening fungal infections has increased dramatically, making fungal infections now responsible for nearly 40% of all deaths from hospital-acquired infections. Increased numbers of patients with an impaired immune system (such as due to ageing, severe burns, AIDS, chemotherapy against cancer, or immunosuppressive therapy for organ transplants), together with a growing list of potential pathogenic fungi are recognized as factors contributing to this rising public health-hazard. There is only a limited set of anti-fungal compounds available, and resistance to existing anti-fungal drugs is becoming a problem of increasing concern. Also clinically used antimycotics may show harmful side effects.

Fungi are also responsible for substantial economic losses due to food spoilage caused by highly dangerous toxins (mycotoxins). To add to this problem food additives to prevent fungal contamination may also be potentially carcinogenic. Additionally plant pathogenic fungi cause huge crop losses and this has promoted the extensive use of pesticides all over the world. Some pesticides have deleterious effects on other organisms than the pests they are intended to control, on water quality, and on the environment in general.

In order to efficiently fight fungi, it is imperative that an anti-fungal inhibits an essential function of the fungus. Currently, treatment of fungal infections depends on the use of fungicides that target the fungal membrane. The specificity and therefore the utility of these compounds is limited because the differences between mammalian and fungal membranes are moderate. Much of the structure, physiology and metabolism of fungal and animal cells is highly conserved. This conservation in cellular function has made it difficult to find agents that selectively discriminate between pathogenic fungi and their human hosts. Consequently, current antifungals are often not specific enough and as a result can cause serious side effects to patients. In addition, several fungal species exhibit increasing resistance to these fungicides. There is a need to develop new and more advanced anti-fungal drugs with novel modes of action and broad spectra directed against plant and animal pathogens.

Selective toxicity towards the fungal cell requires interaction with an essential target not found in other eukaryotic cells. Higher specificity can be achieved by identifying anti-fungals, which target a unique function/attribute of the fungus. Fungal cell wall biosynthesis is one possible target for new anti-fungal drugs, since it is essential for fungal viability and does not occur in mammals. The cell wall of fungi is essential not only in maintaining the osmotic integrity of the fungal cell but also in cell growth, division, and morphogenesis. The cell wall is comprised of glycoproteins and several carbohydrate polymers, namely chitin, beta-1,3-glucan, alpha-1,3 glucan and beta-1,6-glucan [Smits et al., (1999). *Current Opinion In Microbiology* 2: 348-352]. Beta-1,6-glucan is a component of fungal cell walls, where it occurs as a polymer covalently attached to glycoproteins and to other cell wall structural polymers such as beta-1,3-glucan and chitin. It acts as an adhesive covalently linking glycosylated glycoproteins with the cell wall beta-1,3-glucan and chitin polymers in a cross linked extracellular matrix [Lussier *et al.,* (1998). *Proc. Natl. Acad. Sci:* 95: 9825-9830]. Cell wall beta-1,6-glucan is widespread amongst several pathogenic fungi occurring in both ascomycetes and basidiomycetes (including both plant and animal pathogens) for example *Candida albicans, Candida glabrata, Cryptococcus neoformans, Erysiphe graminis, E. cichoracearum, Sphaerotheca fuliginea, Podosphaera leucotricha, Uncinula necator, Ruccinia, Rhizoctonia, Ustilago, Venturia inaequalis, Helminthosporium, Septoria rodorum, Botrytis cinerea, Cercospora arachidicola, Pseudo-cercosporella herpotrichoides, Pyricularia oryzal,* *Phytophthora infestans, Eusarium, Verticillium, Plasmopara and Alternaria.* It is essential for viability [Nagahashi *et al.,* (1998): *Journal of Bacteriology* 180 (19): 5020-5029; Lussier *et al.* (1998): *Proc.Natl. Acad. Sci.* 95: 9825-9830].

Mature beta-1,6-glucan molecules are mainly found on the outside of the skeletal framework and interconnect a particular class of cell wall proteins (CWPs), glycosyl phosphatidylinositol (GPI)-CWPs, with the framework [Smits et al., (1999): *Current Opinion In Microbiology* 2: 348-352]. The GPI-CWPs are indirectly linked to the beta-1,3-glucan network via a highly branched and water-soluble β1,6-glucan molecule which in turn is attached to a remnant of the GPI anchor. This structural complex of GPI-CWP→β1,6-glucan→β1,3-glucan has been extensively investigated. The external protein layer (CWPs) play a critical role in restricting permeability of the wall, and limit access of antifungal peptides to their target, the plasma membrane.

CWPs showing a similar pattern of beta-glucosylation as GPI-CWPs have been extracted from several *Ascomycete* fungi such as *Aspergillus, Paecilomyces, Penicillium, Candida, Fusarium, Hansenula,* and *Wangiella.* Indeed, genes encoding putative GPI-CWPs have been cloned from the human pathogens *Candida albicans, Candida glabrata, Coccidioides immitis* and *Penicillium marneffei* as well as from the plant pathogens *Fusarium oxysporum* and *Glomerella cingulata.* Several of the GPI-CWPs from *C. albicans* and *C. glabrata* mediate attachment to epithelial cells on the host surface and thus are involved in virulence of these pathogenic fungi.

The fungal wall, as an essential organelle that is absent from mammalian cells, forms an ideal target for new antifungals. Compounds inhibiting chitin and beta-1,3-glucan synthesis indeed act as specific anti-fungals. These compounds have been identified by virtue of the availability of *in vitro* assays for determining the activity of chitin and beta-1,3-glucan synthase. This is well illustrated by anti-fungals of the echinocandin family, which inhibit beta-1,3-glucan synthesis. There are as yet no anti-fungal drugs directed against beta-1,6-glucan synthesis.

The invention provides a method for identifying a substance capable of interacting with beta-1,6 glucan synthase comprising contacting a substrate for beta-1,6 glucan synthase with a material comprising beta-1,6 glucan synthase activity in the presence of said substance under appropriate reaction conditions for said synthase and determining whether beta-1,6 glucan synthase activity is affected by the presence of said substance. The invention further provides a method for identifying a substance capable of interacting with beta-1,6 glucan synthase comprising contacting a substrate for beta-1,6 glucan synthase with a material comprising beta-1,6 glucan synthase activity in the presence of said substance under appropriate reaction conditions for said synthase and determining whether the production of beta-1,6 glucan is affected by the presence of said substance.

A substance as used herein can be any physical entity (e.g. molecule, compound etc.), derived from either a synthetic or natural source. Preferably said substance can interact with beta-1,6 glucan synthase and as a result of this interaction beta-1,6 glucan synthase activity is altered. The term altered as used herein means that said substance is capable of modulating the activity of beta-1,6 glucan synthase by increasing (enhancing) or decreasing (inhibiting) said synthase activity. More preferred is that said substance can act as a beta-1,6 glucan synthase inhibitor (e.g. can reduce or eliminate beta-1,6 glucan synthase activity). A change in beta 1,6 glucan synthase activity is reflected by a corresponding change in the amount of beta-1,6 glucan synthesised. The material of choice for assay is one in which at least in part beta-1,6 glucan synthase activity is associated with such as for example membrane extracts, cell wall fractions, total cell protein extracts etc. A suitable substrate for the assay is any glucose donor (e.g. UDP-glucose etc.). The invention provides for carbon sources which may serve as a substrate for beta-1,6 glucan synthase such as for example glucose, galactose, sucrose, mannose, maltose, fucose, fructose, sorbitol, glycerol etc.

In a preferred embodiment of the invention said substance capable of interacting with beta-1,6 glucan synthase comprises fungicidal activity. Fungicidal activity herein refers to the ability of said substance to act as an anti-fungal agent. An anti-fungal agent as used herein refers to a substance which can at least in part suppress or inhibit or restrict the growth of a pathogenic organism (e.g. fungi, bacteria etc.). Preferably, said substance capable of interacting with beta-1,6 glucan synthase is capable of decreasing or at least in part inhibiting beta-1,6 glucan synthase activity.

In a preferred embodiment said material comprising beta-1,6 glucan synthase activity is membrane derived. Preferably, said material is an extract from microsomal membranes. Microsomal membranes as used herein are fragmented pieces of endoplasmic reticulum, golgi apparatus and plasma membrane. Even more preferred said microsomal membranes are yeast derived. A distinct advantage of using microsomal membranes, is that it overcomes access to substrate problems caused by limited permeability of the fungal cell wall. In another preferred embodiment said substrate comprises a glucose donor for example glucose and/or galactose. Preferably said appropriate reaction conditions comprise a pH between 6.0-7.0. The pH optimum for beta-1,6 glucan synthase is around pH 6.5, which differs considerably from the pH optimum for beta-1, 3 glucan synthase which was found to be pH 7.5-8.0.

The invention further provides a method for assaying beta-1,6 glucan synthase activity comprising contacting a sample, under appropriate reaction conditions for a beta-1,6 glucan synthase, with a beta-1,6 glucan synthase substrate and determining the production of beta-1,6 glucan. A sample as used herein can be selected from a group consisting of food products (e.g. pastries, sauces etc.) or non-food products (e.g. leather goods etc.), industrial products (e.g. building materials etc.), plant materials (e.g. commercial crops, ornamental crops etc.) and animal materials (e.g. canine, pharmaceutical samples including human diagnostic materials etc.). The sample supplied for assay can be in the form of an homogenous mixture, tissue sample, swab sample etc. Preferably said sample is a material suspected of comprising a fungus.

In a preferred embodiment of the invention the production of beta-1,6 glucan is assayed using an inhibition Immunoassay. Typical immunoassay formats known in the art often include the use of specific binding agents, such as antibodies, or ligand receptors or other binding proteins. The following combinations are known; carrier proteins and their binding haptens, receptor proteins and their ligands, antibodies and their antigens. A preferred embodiment of the present invention is that the assay is carried out using a competition assay format, more preferably using an inhibition enzyme Immunoassay. The inhibition Immunoassay as used herein the invention refers to a technique that uses an antigen (analyte) and antibody format to determine the relative amounts of newly synthesised analyte. The analyte as used herein refers to beta-1,6 glucan. The invention provides for all assay formats, involving specific binding relations between the analyte (beta-1,6 glucan) to be detected or determined and a detecting or determining agent. Methods to detect a reaction product are known in the art. These procedures include, but are not limited to protein bioassay or immunoassay techniques ELISA, bead assays, surface plasma resonance (SPR) based assays and proteomic technologies. Present day methods for detecting and determining the synthesis of fungal cell wall structural polymers such as beta-1,-3 glucan and chitin are not applicable for beta-1,-6 glucan. The existing beta-glucan product isolation and detection methods do not permit the detection of a beta, 1-6 glucan reaction product. Glucans (e.g. beta-1,3-glucans) are typically detected with methods which involve several steps; radioactive labelling, precipitation, filtration, and detection of radiolabelled product steps. The precipitation step is normally carried out using trichloroacetic acid (TCA) which precipitates insoluble carbohydrate products which are then retained by filtration through a glass fibre filter. In the present invention it was observed that beta-1,-6 glucans are not precipitated by TCA and as a result do not remain on a glass-fibre filter and thus cannot be detected by the existing beta-glucan detection methods. Instead the beta-1,-6 glucans are precipitated with ethanol and not trichloroacetic acid (TCA) precipitated, and detected using an inhibition Immunoassay.

In one aspect the invention provides an inhibition enzyme Immunoassay, wherein said inhibition enzyme Immunoassay comprises coating a body with beta-1,6-glucan and providing said body with at least part of said sample, said assay further comprising providing an antibody with binding specificity for beta-1,6-glucan and detecting synthesized beta-1,6-glucan by deducing the amount of antibodies bound to said body. By way of example of an inhibition enzyme Immunoassay, a body (e.g. a 'solid surface' such as a micro-titer plate or any other object) is coated with pustulan (a commercial beta-1,6-glucan) or any molecule comprising at least beta-1,6-glucan or a beta-1,6-glucan-conjugate, blocked and then washed. Blocking reagents are well known in the art (e.g. example gelatin). A sample as used herein refers to sample comprising steady state synthesised beta-1,-6 glucan and/or beta-1,6-glucan being transiently synthesised or a control sample. A control sample as used herein refers to a beta 1,6-glucan standard (e.g. pustulan standard). The sample is combined with antibodies directed against beta-1,6-glucan. Dependent on the amount of newly synthesised beta-1,6-glucan, there is competition for antibody binding between the coated beta-1,6-glucan (for example pustulan) and the newly synthesised beta-1,6-glucan. Subsequently the wells of the micro-titre plate are washed, and the amount of antibodies bound to the pustulan coating can be quantified by adding a second antibody that is enzyme labelled (by covalent coupling) and raised against the first antibody. Normally assays of the kind include a label, often a visually detectable or direct label (e.g. radioactive label, enzyme label, fluorescent label chemiluminescent label, bioluminescent label, gold label etc.). Examples of commonly used enzyme labels for immunoassays are horseradish peroxidase, alkaline phosphatase and β-galactosidase etc.. Other methods to detecting binding or absence of binding between analyte and binding partners have been disclosed in the art. The amount of product formed (newly synthesized beta, 1,6 glucan) an indirect measure of beta, 1-6 glucan synthase activity can be determined with a given reagent (e.g. for a horseradish peroxidase label suitable substrates are o-phenylenediamine, diaminobenzidine, chloronaphthol, aminoethylcarbazole etc.). Suitable enzyme label-substrate combinations are known and methods of their detection are known. The reaction product of horse radish peroxidase enzyme label o-phenylenediamine substrate combination can be measured by reading the optical absorbance of the oxidation product at a specific wavelength (OD490nm). The optical absorbance of the reaction product (e.g. signal) can be measured using a spectrophotometer (for example using a microtiter plate reader (SpectraCount® (Packard Instrument Company, Inc., Meriden, CT 06450 U.S.A)). Low levels of newly synthesised beta-1,6-glucan result in a high signal (e.g. more secondary antibodies bound to the coated pustulun) and high levels of newly synthesised beta-1,6-glucan likewise result in a low signal (e.g. less secondary antibodies bound to the coated pustulun).

In a preferred embodiment said inhibition Immunoassay comprises contacting a reaction product, obtained by said contacting of a beta-1,6 glucan synthase substrate with said synthase in the presence of said substance, with beta-1,6 glucan coated onto a solid surface and antibody with binding specificity for beta-1,6 glucan and determining antibody bound to said solid surface coated with beta-1,6 glucan and/or antibody not bound to said solid surface coated with beta-1,6 glucan. Preferably an enzyme labelled antibody with binding specificity for the beta-1,6 glucan specific antibody is used to determine the same. In a preferred embodiment an inhibition enzyme Immunoassay as used herein involves the use of antibodies or fragments or derivatives thereof. A derivative of an antibody is any molecule which has similar or the same binding characteristics as the original antibody, but has been changed in other ways. A fragment of an antibody may be any fragment which retains sufficient binding affinity for the analyte. An antibody as used herein is specifically directed against beta- 1,6-glucan and may be monoclonal or a polyclonal antibody. Ways of obtaining antibodies or derivatives and/or fragments thereof are all standard techniques in the art. In addition, instead of the primary antibodies one may use a lectin or any other beta-1,6-glucan binding compound. The invention provides for all known and newly developed assay formats that will allow for the detection and quantification of beta-1,6 glucan comprising specific binding relations between an analyte (beta-1,6 glucan) to be detected or determined and a detecting or determining agent. It is recognized that the present invention provides a person skilled in the art with the tools to design many new assay formats for detecting the possible presence or determining the amount present of newly synthesised analyte (i.e beta-1,6 glucan). The inhibition enzyme Immunoassay method as used herein the invention allows for the detection of soluble and insoluble beta-1,6-glucan. A preferred embodiment of the present invention is that the synthesized beta-1,6-glucan comprises a water-soluble β1,6-glucan molecule. The invention further provides a method for quantifying beta-1,6 glucan synthesised in a sample. By way of example samples comprising a standard amount of pustulan (serial dilutions) and a control can be used to quantify the amount of beta, 1-6 glucan equivalents in a sample. The absorbance of the reaction product produced by a serial dilution of the beta 1-6 glucan equivalents (e.g. pustulun) can be used to generate a calibration curve on a spectrophotometer using the enzyme inhibition immunoassay format as disclosed herein the invention. This calibration curve can then be used to extrapolate the amount of newly synthesised beta-1, 6 glucan in any given sample.

The invention provides a method according to the invention, performed in a high throughput setting. The immunoassay as disclosed in the invention can be used routinely for large scale sample analysis and in a high throughput setting for the rapid identification of a substance capable of interacting with beta-1,6 glucan synthase and altering its activity. Preferably said substance is a beta-1,6 glucan synthase inhibitor and can target specifically the integrity of the fungal wall. One main problem associated with the use of current anti-fungals that disrupt the membrane or affect intracellular targets is the limited permeability of the fungal cell wall. To be able to rapidly identify a substance (e.g. a fungal cell wall perturbing biomolecule) capable of interacting with beta-1,6 glucan synthase and altering its activity is a distinct advantage of the present invention. Fungal cell wall perturbing biomolecules identified by said assay can be readily used to increase the effectiveness of known fungal membrane perturbing biomolecules (e.g. osmotin etc..), when used in combination.

The invention further provides for a substance capable of interacting with beta-1,6 glucan synthase obtainable by a method according to the invention. A preferred embodiment is a substance which can act as a beta-1,6 glucan synthase inhibitor (e.g. a substance that can reduce or eliminate beta-1,6 glucan synthase activity). The enzyme inhibition immunoassay as disclosed herein the invention provides the means to identify natural and more fungal-specific inhibitors. Preferably said substance can control or reduce the risk of systemic infections caused by both plant and animal pathogenic organisms (e.g. fungi, bacteria etc.). More preferred is that said substance has fungicidal activity.

The invention further provides a composition comprising a substance capable of interacting with beta-1,6 glucan synthase obtainable by a method according to the invention and a carrier. Suitable basis for preparation of compositions are known, and they may be in dosage forms such as tablets, pills, powders, suspensions, capsules, suppositories, injection preparations, ointments, eye drops etc.. Preferably said composition is in a form that can be administered to a plant, animal (including human), food or non-food product, industrial product etc. as an anti-fungal agent. Preferably said carrier is a pharmaceutically acceptable carrier (e.g. drug carrier system) or inert carrier. A preferred embodiment is that said composition is a pharmaceutical composition.

The invention further provides for a substance capable of interacting with beta-1,6 glucan synthase obtainable by a method according to the invention for use as an anti-fungal agent. The invention further provides for use of said substance as an anti-fungal agent to treat or prevent a fungal contamination or fungal infection. A preferred embodiment is that said substance has food grade status and thus suitable for human consumption or external application. It is understood that said substance can be used alone or in combination with other anti-microbial or anti-fungal substances such as for example for food product (e.g. food, pastries, sauces etc.), and non-food product (e.g. detergents, creams, ointments etc.) and pharmaceutical applications. This will ensure broad spectrum resistance against pathogenic agents, and also enhance the shelf life/preservation of food and non-food products.

The invention provides for use of a substance which has fungicidal activity together with at least one other compound having fungicidal activity to augment or supplement said fungicidal activity. For example said substance can be used in synergy with:-
a) cell wall degrading/hydrolytic enzymes [e.g. molecules that inhibit beta 1,3-glucan or chitin synthesis or compounds that interfere with incorporation of cell wall proteins or compounds that bind to particular cell wall components and which interfere with normal cell wall assembly or any molecule directed against fungal cell wall construction] and/or
b) cell wall perturbing biomolecules and/or
c) membrane active compounds [e.g. any molecule directed against fungal plasma membrane] and/or
d) antibiotics and/or
e) anti-microbial and anti-fungal agents from synthetic or natural sources (e.g. antimicrobial peptides, chitosan).
One preferred embodiment of the invention is a substance capable of interacting with beta-1,6 glucan synthase and altering its activity obtainable by a method according to the invention, which can act synergistically with at least one other compound. The use of synergistic combinations of antimicrobial or anti-fungal agents has many advantages. One such advantage is that it minimises the known risk associated with the use of potentially deleterious anti-fungal agents which can be used in lower dosages to achieve the same effect. It also lowers risks associated with the use of non specific/non-selective anti-fungal agents. In addition certain food preservative processes cause damage to textural and flavour properties of food products. The use of said substance alone or in synergistic combination with other anti-fungal or anti-microbial agents, can reduce and even eliminate the need for damaging food heat preservation treatments. In addition said substance can be used as an anti-fungal agent for crop treatment programs to reduce or eliminate the use of harmful pesticides/biocides [e.g. spray treatments]. It can be incorporated into products as an anti-fungal agent [e.g. household materials, detergents, food products etc.] or applied to products [e.g. as an external coating to leather products etc.] to reduce risk of fungal spoilage or fungal contamination.

The invention further provides a method to determine whether said substance comprises fungicidal activity comprising incubating a sample comprising a fungus, under conditions allowing for growth of said fungus, providing said sample with said substance or said substance with at least one other compound comprising fungicidal activity, and determining whether growth of said fungus is at least in part inhibited. The invention further provides use of beta-1,6 glucan synthase for detection of a fungus. The sample supplied for assay can be in the form of an homogenous mixture, tissue sample, swab sample etc.

The invention provides a method for the high throughput screen of food and non food products, crops, medical samples to detect a pathogenic agent [e.g. fungal contaminant]. The present invention can aid in the detection of an early pathogenic fungal contamination and will also provide the means to assist in its treatment.

The invention provides for use of a substance capable of interacting with beta-1,6 glucan synthase according to the invention for the preparation of a medicament for at least in part preventing or treating a fungal infection. Preferably said substance capable of interacting with beta-1,6 glucan synthase is at least in part a beta-1,6 glucan synthase inhibitor. Suitable basis for preparation of a medicament and methods for administrating said medicament to a recipient are known. For example the medicament can be in the form of an ointment or an anti-fungal cream for external application. Optimal dosage and modes of administration can readily be determined by conventional protocols.

The invention provides for a method of treatment or prevention of a fungal infection of an animal or human being comprising administering a substance or a composition comprising said substance according to the invention to said animal or human being. The invention provides a method of treatment or prevention of a pathogenic infection comprising administering a composition comprising a substance capable of interacting with beta-1,6 glucan synthase to a suitable recipient [animal or human being]. Suitable basis for preparation of said compositions and methods for administrating said compositions to a suitable recipient are known.

The invention provides for a method of treatment of a fungal contamination of a plant comprising administering a substance or a composition comprising said substance according to invention to said plant. Preferably said plant is a commercial crop, ornamental crop etc.. Modes of administration can readily be determined by conventional protocols and may take the form of sprays, dissoluble pellets etc.

The invention provides for a method of treatment of a fungal contamination comprising administering a substance or a composition comprising said substance according to the invention to said food or non-food product. Preferably said substance is administrated to a food or non-food product as a preservative.

The invention provides a kit comprising at least one means for performing a method as disclosed in the invention. The invention provides the basis for a kit for a high throughput screen for beta-1,6-glucan synthase inhibitors and for the diagnosis of a fungal infection or fungal contamination.

### Examples

### Example 1. Isolation of microsomal membranes.

Exponential growing yeast cells were used to prepare a membrane extract, to which (at least part of) the beta-1,6-glucan synthase activity is associated. Yeast cells are grown to early log phase (200 ml culture to OD₆₀₀ₙₘ= 0.5 - 1.0). Cells are harvested and washed in ice-cold buffer A (50 mM Tris-Cl pH 7.5, 1 mM 2-mercaptoethanol, 1 mM EDTA). Cells are transferred to a screwcap tube and resuspended in 100 µl buffer A. Cells are broken with glassbeads in a FastPrep FP120 (Savant; Bio101) for 15 sec at speed 6. Cell debris and glassbeads are removed by low-speed centrifugation (5,000 x g, 5 min at 4°C). Microsomal membranes were isolated by 36,000 x g centrifugation for 30 min at 4°C, and resuspended in 50 mM Tris-Cl pH 7.5, 1 mM 2-mercaptoethanol, 1 mM EDTA, 33% glycerol and stored at -70°C.

### Example 2. Beta-1,6-glucan synthase assay.

The isolated membrane extract was incubated using the following reaction conditions.
The assay mixture consists of 50 - 100 µg protein, 50 mM UDP-glucose, 50 mM 2-(n-morpholino)ethanesulphonic acid (MES) pH 6.5, 2.1 mM EDTA, 4.1% glycerol (v/v), 150 µM Guanosine 5'-TriPhosphate (GTP), and 0.75% BSA. The reactions are carried out at 30°C and, although linearity over time has to be studied more precisely, a signal can be clearly detected after 30 min. The reactions are stopped by the addition of 500 µl ethanol (absolute). Reaction products are precipitated for ≥ 60 min at 0°C and centrifuged at 14,000 rpm for 10 min at 4°C (Eppendorf 5402), and thereafter the pellet is resuspended in PBS. Detection of the reaction product [*in-vitro* synthesized beta-1,6-glucan] is performed with an inhibition Enzyme ImmunoAssay (EIA).

### Example 3. Inhibition EIA for quantification of beta-1,6-glucan.

Each well of a microtiter plate (Greiner 655 092) is coated overnight at 4°C with 2 µg/ml pustulan in PBS pH 7.0 [Pustulan (commercial Beta-1,6 glucan) or any beta-1,6-glucan molecule or beta-1,6-glucan-conjugate may be used in the inhibition EIA]. The plate is then washed three times with 300 µl PBS pH 7.4 containing 0.05% (v/v) Tween 20 (PBT), and subsequently blocked with 300 µl PBT containing 0.5% gelatin (PBTG) for at least 30 min on a shaker. After removal of the blocking solution, the plate is washed again as described above. A pustulan standard or a sample [*in-vitro* synthesized beta-1,6-glucan] in a volume of 100 µl (in PBS pH 7.4) is put in a well, and then mixed with 100 µl of anti-beta-1,6-glucan antibodies (1:100,000 in PBTG) and incubated by shaking for 1 h. The plate is then washed again thoroughly, and each well is incubated with 200 µl goat-anti-rabbit antibodies labeled with horseradish peroxidase (1:5,000 in PBTG) for 1 h shaking. Again, the plate is washed three times and each well is incubated for 10 min with 200 µl *o*-phenylenediamine (2 mg/ml in 50 mM citrate-phosphate buffer pH 5.5, with 0.015% H₂O₂). This reaction is stopped by the addition of 50 µl of 2 M HCl, and the OD₄₉₀ₙₘ is read with a SpectraCount® (Packard Instrument Company, Inc., Meriden, CT 06450 U.S.A.) platereader. Pustulan standards in 9 serial dilutions (1,000 - 3.9 ng/ml in PBS pH7.4) and a control containing no pustulan are loaded in quadruplo. This data renders a calibration curve, which can be used for interpolating the OD₄₉₀ₙₘ of the samples. Using the five-parameter curve-fitting function of the I-Smart software (Packard Instrument Company, Inc., Meriden, CT 06450 U.S.A.), the amount of pustulan equivalents in each sample can be quantified.

### Legends

### Figure 1.

### Detection of beta-1,6 glucan in a sample with an inhibition enzyme immunoassay.

Detection of the reaction product [*in-vitro* synthesised beta-1,6-glucan] is performed with an inhibition enzyme Immunoassay (EIA).
a) Micro-titre plates are coated with pustulan (a commercial beta-1,6-glucan) and then blocked.
b) *In vitro* synthesised beta-1,6-glucan in combination with antibodies directed against beta-1,6-glucan is added. Dependent on the amount of beta-1,6-glucan, there is competition for binding the antibodies between the coated pustulan and the *in vitro* produced beta-1,6-glucan.
c) The wells are washed, and the amount of antibodies bound to the pustulan coating can be quantified by adding a second antibody that is enzyme-labelled and raised against the first antibody. The amount of enzyme activity can be determined with a given reagent, which can be detected with a micro-titre plate reader. Low levels of beta-1,6-glucan in the sample result in a high signal, and high beta-1,6-glucan levels in a low signal.

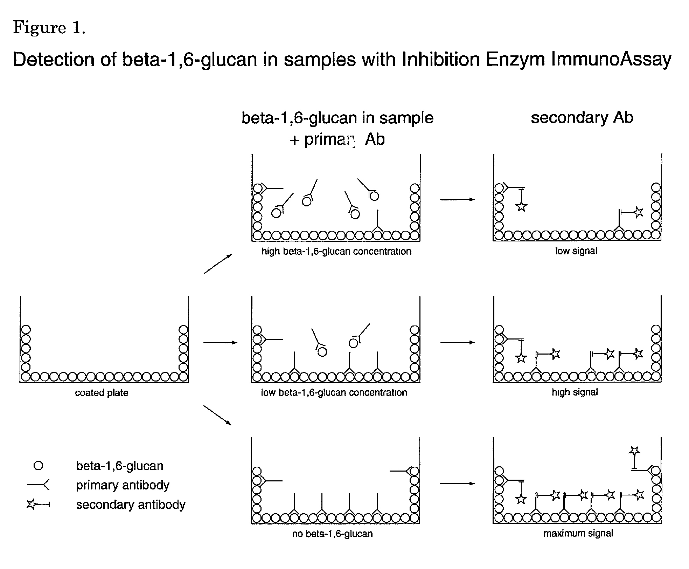

## Claims

1. A method for identifying a substance capable of interacting with beta-1,6 glucan synthase comprising contacting a substrate for beta-1,6 glucan synthase with a material comprising beta-1,6 glucan synthase activity in the presence of said substance under appropriate reaction conditions for said synthase and determining whether beta-1,6 glucan synthase activity is affected by the presence of said substance.

2. A method according to claim 1 wherein it is determined whether the production of beta-1,6 glucan is affected by the presence of said substance.

3. A method according to claim 1 or 2 wherein said substance capable of interacting with beta-1,6 glucan synthase comprises fungicidal activity.

4. A method according to anyone of claims 1-3 wherein said substance capable of interacting with beta-1,6 glucan synthase is capable of decreasing beta-1,6 glucan synthase activity.

5. A method according to anyone of claims 1-4 wherein said material comprising beta-1,6 glucan synthase activity is membrane derived.

6. A method according to claim 5 wherein said material is an extract from microsomal membranes.

7. A method according to claim 6 wherein said microsomal membranes are yeast derived.

8. A method according to anyone of claims 1-7 wherein said substrate comprises a glucose donor.

9. A method according to anyone of claims 1-8 wherein said appropriate reaction conditions comprise a pH between 6.0-7.0.

10. A method for assaying beta-1,6 glucan synthase activity comprising contacting a sample, under appropriate reaction conditions for a beta-1,6 glucan synthase, with a beta-1,6 glucan synthase substrate and determining the production of beta-1,6 glucan.

11. A method according to claim 10 wherein said sample is a material suspected of comprising a fungus.

12. A method according to claim 11 wherein said material is selected from a group consisting of food products, industrial products, plant materials and diagnostic samples.

13. A method according to anyone of claims 1-12 wherein the production of beta-1,6 glucan is assayed using an inhibition Immunoassay.

14. A method according to claim 13 wherein said inhibition Immunoassay comprises contacting a reaction product, obtained by said contacting of a beta-1,6 glucan synthase substrate with said synthase in the presence of said substance, with beta-1,6 glucan coated onto a solid surface and antibody with binding specificity for beta-1,6 glucan and determining antibody bound to said solid surface coated with beta-1,6 glucan and/or antibody not bound to said solid surface coated with beta-1,6 glucan.

15. A method according to claim 14, wherein an enzyme labelled antibody with binding specificity for the beta-1,6 glucan specific antibody is used to determine the same.

16. A method according to any one of claims 1-15 performed in a high throughput setting.

17. A substance capable of interacting with beta-1,6 glucan synthase obtainable by a method according to anyone of claims 1-16.

18. A composition comprising a substance according to claim 17 and a carrier.

19. A composition according to claim 18 which is a pharmaceutical composition.

20. A substance capable of interacting with beta-1,6 glucan synthase obtainable by a method according to anyone of claims 1-16 for use as an anti-fungal agent.

21. Use of a substance according to claim 17 which has fungicidal activity together with at least one other compound having fungicidal activity to augment or supplement said fungicidal activity.

22. Use of a substance capable of interacting with beta-1,6 glucan synthase according to claim 17 for the preparation of a medicament for at least in part preventing or treating a fungal infection.

23. A method of treatment or prevention of a fungal infection of an animal or human being comprising administering a substance according to claim 17, or a composition comprising said substance according to claim 18 or claim 19 to said animal or human being.

24. A method of treatment of a fungal contamination of a plant comprising administering a substance according to claim 17, or a composition comprising said substance according to claim 18 to said plant.

25. A method of treatment of a fungal contamination of a food or non-food product comprising administering a substance according to claim 17, or a composition comprising said substance according to claim 18 to said food or non-food product.
